# EUROPEAN PATENT APPLICATION

(11) **EP 4 681 668 A1**
(43) Date of publication of application: **21.01.2026**
(21) Application number: 23938130.4
(22) Date of filing: 10.08.2023
(51) Int. Cl.: A61B 18/12, A61B 18/14, A61B 18/18, A61B 18/00

(54) **ABLATION NEEDLE ASSEMBLY AND ABLATION SYSTEM**

(30) Priority: 24.05.2023 CN 202310588817
(71) Applicant: Zhejiang Curaway Medical Technology Co., Ltd., Hangzhou, Zhejiang 310018 (CN)
(72) Inventor: CHEN, Qiang, Hangzhou, Zhejiang 310018 (CN); JIANG, Tianan, Hangzhou, Zhejiang 310018 (CN); LIN, Minqin, Hangzhou, Zhejiang 310018 (CN); CAI, Jiang, Hangzhou, Zhejiang 310018 (CN); WANG, Zhiqing, Hangzhou, Zhejiang 310018 (CN); SHAO, Wenyu, Hangzhou, Zhejiang 310018 (CN); ZHU, Yangli, Hangzhou, Zhejiang 310018 (CN)
(74) Representative: Argyma
(86) International application number: PCT/CN2023/112117
(87) International publication number: WO 2024/239461

(57) **Abstract**

The present invention discloses an ablation needle assembly and an ablation system. The ablation needle assembly includes a hollow insulated coaxial outer tube and an ablation needle, the ablation needle including an ablation needle body and an ablation handle connected to the proximal end of the ablation needle body. The ablation needle body movably passes through the interior of the insulated coaxial outer tube, the proximal end of the insulated coaxial outer tube being detachably connected to the distal end of the ablation handle; and a portion of the distal end of the ablation needle body extending out of the insulated coaxial outer tube is tightly fitted with the uninsulated portion at the distal end of the insulated coaxial outer tube to form a single electrode. By providing a detachable connection between the ablation handle and the insulated coaxial outer tube, after the initial needle insertion, the insulated coaxial outer tube serves as a re-entry needle channel, avoiding secondary injury to tissue during secondary needle insertion. By tightly fitting the portion of the ablation needle body extending out of the insulated coaxial outer tube with the uninsulated portion of the insulated coaxial outer tube to form a single electrode, interference current generated outside the lesion is avoided, thereby preventing ablation damage to normal tissues.

## Description

### Technical Field

The present invention relates to the technical field of medical devices, and more particularly to an ablation needle assembly and an ablation system.

### Background Art

In the prior art, for tumor diagnosis of liver, kidney, soft tissue, etc., a biopsy needle can penetrate into the lesion site, and acquire a small amount of tissue at a part of the lesion site for pathological analysis. For the treatment of such lesion sites, it is feasible to insert a radio frequency ablation needle into the lesion, and use radio frequency energy to generate high temperature in the local tissue of the lesion, so as to make the lesion tissue coagulative necrosis to achieve the purpose of treatment.

As a minimally invasive interventional therapy device, the radio frequency ablation needle is mainly used for the treatment of tumors in the liver, kidney, soft tissue and other parts, and the existing ablation needles are mostly integrated. On the one hand, if other operations such as biopsy are needed before and/or after ablation, multiple punctures are needed, which is difficult and aggravates the damage to tissues. If lung nodules need to be pre-ablated before biopsy sampling, and if it is a highly suspected malignant liver tumor under imaging, immediate ablation treatment after the biopsy will be considered. On the other hand, the second electrode may be generated on the needle body outside the working area of the radio frequency ablation needle, the interference current may be generated on the second electrode, and ablation damage may be caused to normal tissues outside the target ablation area.

### Summary of the Invention

To address the aforementioned technical issues, the present invention provides an ablation needle assembly and an ablation system. By enabling a detachable connection between the ablation handle and the insulated coaxial outer tube in the ablation needle assembly, after an initial needle insertion, the insulated coaxial outer tube serves as a re-entry needle channel, thereby avoiding secondary injury to tissue during a second needle insertion. Through tight fitting between the ablation needle body and the insulated coaxial outer tube, a portion of the distal end of the ablation needle body extending out of the insulated coaxial outer tube, together with the cutting edge of the uninsulated portion at the distal end of the insulated coaxial outer tube, forms a single electrode.

This configuration prevents interfering radio frequency currents from being generated outside the lesion area, thereby avoiding ablation damage to normal tissues.

In order to achieve the above object, the technical solution of the present invention is as follows:
an ablation needle assembly, comprising an insulated coaxial outer tube and an ablation needle, wherein the ablation needle comprises an ablation needle body and an ablation handle connected to a proximal end of the ablation needle body, the ablation needle body movably passes through an interior of the insulated coaxial outer tube, and the proximal end of the insulated coaxial outer tube is detachably connected to the distal end of the ablation handle;
and the ablation needle body is closely fitted with the insulated coaxial outer tube, and the portion of the distal end of the ablation needle body protruding out of the insulated coaxial outer tube and the portion of the distal end of the insulated coaxial outer tube without insulation form a single electrode.

According to a preferred implementation mode of the present invention, the uninsulated portion of the distal end of the insulated coaxial outer tube transitions to the ablation needle body to form a cutting edge, and the cutting edge is arranged to effectively reduce the puncture force of the ablation needle body when it is punctured.

According to a preferred implementation mode of the present invention, the ablation handle is an ablation handle having an adjustable axial length, the axial length of the ablation handle is adjusted to adjust the distance between the insulated coaxial outer tube and the ablation handle, and the ablation needle body is held stationary to achieve real-time adjustment of the length of the distal end of the ablation needle body extending out of the insulated coaxial outer tube.

Through the above implementation mode, during adjustment of the axial length of the ablation handle, the distance between the insulated coaxial outer tube and the ablation handle is adjusted while the ablation needle body remains stationary, thereby inversely adjusting the length by which the distal end of the ablation needle body extends beyond the insulated coaxial outer tube, i.e., the length of the electrode working area of the ablation needle, so that the ablation needle can adapt to the ablation of lesions of different sizes. Moreover, during adjustment of the axial length of the ablation handle, the ablation needle body remains stationary, i.e., the length of the ablation needle body remains unchanged, allowing clinicians to predict the clinical working space range of the ablation needle.

In a preferred implementation mode of the present invention, the ablation handle further includes a rotating shaft, wherein the ablation needle body passes through the rotating shaft and is coaxially and movably connected to the rotating shaft, the rotating shaft being coaxially arranged with the ablation handle, and the distal end of the rotating shaft being detachably connected to the proximal end of the insulated coaxial outer tube.

The rotating shaft moves axially to achieve a change in the axial length of the ablation handle.

In a preferred implementation mode of the present invention, the ablation handle further includes a rear needle hub and a hand-held portion, wherein the rear needle hub is detachably connected to the proximal end of the insulated coaxial outer tube; and the distal end of the rotating shaft is fixedly connected to the rear needle hub, and the proximal end of the rotating shaft is connected to the hand-held portion.

Axial movement of the rotating shaft can adjust the distance between the distal end of the hand-held portion and the rear needle hub, thereby achieving adjustment of the axial length of the ablation handle.

In a preferred implementation mode of the present invention, the proximal end of the rotating shaft is threadedly connected to the hand-held portion, and the rotating adjustment of the rotating shaft causes axial movement of the rotating shaft, thereby adjusting the distance between the rear needle hub and the distal end of the hand-held portion, and thus adjusting the length of the ablation handle.

In a preferred implementation mode of the present invention, the hand-held portion comprises an adjustable rotary cap provided at the distal end, a cavity, and a fixed seat fixedly connected to the cavity, and a proximal end of the rotating shaft is threaded with the interior of the cavity;
and rotating the adjustable rotary cap causes the rotating shaft and the adjustable rotary cap to rotate synchronously, thereby causing axial movement of the rotating shaft to adjust the distance between the rear needle hub and the distal end of the hand-held portion, and thus adjusting the length of the ablation handle.

In a preferred implementation mode of the present invention, a locking structure is provided at the connection between the adjustable rotary cap and the cavity for locking the rotation of the adjustable rotary cap, wherein when the locking structure is in a locked state, the axial length of the ablation handle cannot be adjusted, and when the locking structure is in an unlocked state, the axial length of the ablation handle can be adjusted, thereby adjusting the length of the electrode working area of the ablation needle body. By adopting the above implementation, risks caused by inadvertent touching of the adjustable rotary cap leading to unintended changes in the length of the electrode working area of the ablation needle can be prevented.

In a preferred implementation mode of the present invention, the adjustable rotary cap and the cavity may be locked via a snap-fit connection, i.e., the locking structure comprises an emboss and a groove.

A plurality of embosses are provided on a proximal end surface of the adjustable rotary cap, and a groove is formed on a distal end surface of the cavity; or
a plurality of grooves are formed on the proximal end surface of the adjustable rotary cap, and an emboss is provided on the distal end surface of the cavity; and
the emboss is embedded in the grooves to limit the rotation of the adjustable rotary cap.
In a preferred implementation mode of the present invention, the locking structure further includes a snap ring and a first limiting unit, and
the proximal end surface of the adjustable rotary cap has a plurality of extending shafts extending toward the cavity, the first limiting unit is provided on the extending shaft, and a snap ring is provided circumferentially inside the cavity; and when the adjustable rotary cap is connected to the cavity, the first limiting unit snaps into one side of the snap ring close to the proximal end, thereby limiting axial movement of the adjustable rotary cap. The above implementation mode can prevent unintended unlocking of the adjustable rotary cap.

In a preferred implementation mode of the present invention, the extending shaft is further provided with a second limiting unit, and when the adjustable rotary cap is separated from the cavity, the snap ring is embedded between the first limiting unit and the second limiting unit, thereby limiting axial movement of the adjustable rotary cap during a movement of the rotating shaft.

In a preferred implementation mode of the present invention, the insulated coaxial outer tube comprises a coaxial outer tube made of metallic material and an insulating layer coated on an outer surface of the coaxial outer tube; and the insulating layer is preferably parylene nanocoating. In a preferred implementation mode of the present invention, the ablation handle is provided with a cooling medium inlet channel and a cooling medium outlet channel which may be specifically provided within the fixed seat at the proximal end of the ablation handle; and the cooling medium inlet channel forms a cooling medium inlet on the side surface of the fixed seat, and the cooling medium outlet channel forms a cooling medium outlet on the side surface of the fixed seat;
a straight line on which the cooling medium inlet channel is located and a straight line on which the cooling medium outlet channel is located respectively intersect with an axis of the ablation needle; and
the side surface of the fixed seat refers to the side surface excluding the proximal end surface of the fixed seat.

Through the above implementation mode, the cooling medium inlet channel and cooling medium outlet channel, which are communicated with the cooling device, are arranged to intersect with the axis of the ablation needle; and compared with conventional configurations where the cooling medium inlet channel and the cooling medium outlet channel are parallel to the axis of the ablation needle, this arrangement further shortens the axial length of the ablation handle, facilitating the operation of the ablation needle in confined spaces.

In a preferred implementation mode of the present invention, the cooling medium inlet channel is in a straight line perpendicular to the axis of the ablation needle; and/or
the straight line where the cooling medium outlet channel is located is perpendicular to the axis of the ablation needle.

In the perpendicular state, the axial length of the ablation handle can be minimized to the maximum extent, and it can be ensured that the cooling medium can smoothly flow into the channel in the ablation needle.

In a preferred implementation mode of the present invention, the ablation needle further comprises a drainage tube coaxially provided within the ablation needle body,
and the fixed seat comprises an ablation needle body fixed seat and a drainage tube fixed seat, wherein the proximal end of the ablation needle body is sealingly and fixedly connected to the ablation needle body fixed seat, and a first cavity is formed at the gap where the proximal end of the ablation needle body and the ablation needle body fixed seat cooperate.

The proximal end of the drainage tube is in sealed and fixed connection with the drainage tube fixed seat, and a second cavity is formed at the gap where the proximal end of the drainage tube and the drainage tube fixed seat cooperate;
and the first cavity is in communication with the cooling medium outlet channel, the cooling medium outlet channel being located within the ablation needle body fixed seat; and the second cavity is in communication with the cooling medium inlet channel, the cooling medium inlet channel being located within the drainage tube fixed seat.

In a preferred implementation mode of the present invention, the drainage tube is provided therein with a second inner cavity extending from the proximal end to the distal end, the second inner cavity forming a second cooling medium flow path, and the second cooling medium flow path being in communication with the second cavity.

In a preferred implementation mode of the present invention, a first inner cavity extending from the proximal end to the distal end is provided within the ablation needle body, and a gap between the first inner cavity and the drainage tube forms a first cooling medium flow path, the first cooling medium flow path being in communication with the first cavity.

In a preferred implementation mode of the present invention, the portion of the ablation needle body extending out of the insulated coaxial outer tube has a cooling cavity inside, the cooling cavity being provided at the distal end of the drainage tube, and the cooling cavity being in communication with both the first cooling medium flow path and the second cooling medium flow path.

In a preferred implementation mode of the present invention, a thermocouple is provided within the drainage tube, the thermocouple being arranged coaxially within the drainage tube, and the thermocouple sequentially passing through the ablation needle body fixed seat and the drainage tube fixed seat to fixedly connect the two, and protruding from the proximal end of the drainage tube fixed seat and extending out from the side surface of the drainage tube fixed seat.

In a preferred implementation mode of the invention, the cooling medium is water or an inert gas. In a preferred implementation mode of the present invention, the ablation needle assembly further comprises a biopsy needle, and the biopsy needle and the ablation needle are alternately inserted into the insulated coaxial outer tube and detachably connected to the insulated coaxial outer tube; and/or
the ablation needle assembly further comprises a puncture needle, and the puncture needle and the ablation needle are alternately inserted into the insulated coaxial outer tube and detachably connected to the insulated coaxial outer tube, with a distal end of the puncture needle extending out of the insulated coaxial outer tube.

In a preferred implementation mode of the present invention, the insulated coaxial outer tube is connected to the ablation handle, the biopsy needle, or the puncture needle via a Luer connection. Based on the same inventive concept, the present invention also provides an ablation system including the ablation needle assembly of the above embodiment, and an energy generating device electrically connected to the ablation needle body of the ablation needle.

In one of the preferred implementation modes, the energy generating device is a radio frequency generator or other high-frequency energy generators.

In one of the preferred implementation modes, a cooling device is provided in communication with the cooling medium inlet and the cooling medium outlet at the proximal end of the ablation handle.

Due to the adoption of the above technical solution, the present invention has the following advantages and positive effects compared with the prior art.

In the present invention, the ablation needle assembly comprises an insulated coaxial outer tube and an ablation needle, wherein the ablation needle comprises an ablation needle body and an ablation handle, the proximal end of the ablation needle body is fixed inside the ablation handle, and the insulated coaxial outer tube is sheathed outside a portion of the ablation needle body exposed outside the ablation handle and is detachably connected to the ablation handle. After the ablation operation is completed, the insulated coaxial outer tube can be detached from the ablation needle, leaving the insulated coaxial outer tube in the tissue, providing a channel for other operations such as biopsy, avoiding repeated puncture, reducing the damage to the tissue, enabling the ablation and other operations to be more convenient and efficient, and simplifying the surgical procedure and reducing the operation time. Furthermore, in the present invention, the insulated coaxial outer tube is closely connected to the ablation needle body, and the portion of the ablation needle body extending out of the insulated coaxial outer tube and the uninsulated portion of the insulated coaxial outer tube form a single electrode, so that the area outside the target ablation area can be free from interference radio-frequency currents, and ablation damage to normal tissues can be avoided.

In an embodiment of the present invention, the ablation handle is designed as a handle with adjustable axial length. By adjusting the axial length of the ablation handle, the distance between the insulated coaxial outer tube and the ablation handle can be adjusted while keeping the ablation needle body stationary. Since the length of the insulated coaxial outer tube is fixed, when the axial length of the ablation handle is adjusted, the length of the portion of the distal end of the ablation needle body extending out of the insulated coaxial outer tube changes inversely, thereby achieving stepless adjustment of the electrode working area length of the ablation needle body. This allows the ablation needle assembly of the present invention to adapt to lesions of different sizes, making it more flexible and effective in use. Further, during the adjustment, the ablation needle body remains stationary, which not only can avoid the risk of leakage in the circulation channel for cooling medium inside the ablation needle, but also allows clinicians to anticipate the working space range, thereby preventing insufficient operational space during surgery.

In an embodiment of the present invention, in the cooling medium circulation channel of the ablation needle body, the cooling medium inlet channel and the cooling medium outlet channel in communication with the external cooling device are arranged to intersect with the axis of the ablation needle, and the cooling medium inlet and the cooling medium outlet are located on the side surface of the ablation handle except the proximal end face. With respect to the technical solution that the cooling medium inlet channel and the cooling medium outlet channel are parallel to the axis of the ablation needle, the length of the ablation handle is greatly shortened, which can greatly facilitate the clinician to operate the ablation needle in a narrow space, and is of far-reaching clinical significance.

### Brief Description of the Drawings

Fig. 1 is a schematic view showing a structure of an ablation assembly according to embodiment 1 of the present invention;
Fig. 2 is an enlarged view 1 of a portion A of Fig. 1;
Fig. 3 is an enlarged view 2 of a portion A of Fig. 1;
Fig. 4 is a schematic structural view of a locking structure of an ablation needle in a locked state according to embodiment 1 of the present invention;
Fig. 5 is a cross-sectional view of Fig. 4;
Fig. 6 is a schematic structural view of a locking structure of an ablation needle in an unlocked state according to embodiment 1 of the present invention;
Fig. 7 is a cross-sectional view of Fig. 6;
Fig. 8 is a schematic view of an adjustable rotary cap and a rotating shaft of an ablation needle according to embodiment 1 of the present invention;
Fig. 9 is a side view of an adjustable rotary cap and a rotating shaft after assembly according to embodiment 1 of the present invention;
Fig. 10 shows the shortest state of an electrode working area of a needle body of an ablation needle according to embodiment 1 of the present invention;
Fig. 11 is an enlarged view of a portion B of Fig. 10;
Fig. 12 shows the longest state of an electrode working area of a needle body of an ablation needle according to embodiment 1 of the present invention;
Fig. 13 is an enlarged view of a portion C of Fig. 12;
Fig. 14 is a schematic view of a cooling medium flow path in an ablation needle body according to embodiment 1 of the present invention;
Fig. 15 is an enlarged view of a portion E of Fig. 14;
Fig. 16 is an enlarged view of a portion F of Fig. 14;
Fig. 17 is an enlarged view of a portion G of Fig. 14;
Fig. 18 is a schematic view of step one of an ablation assembly of embodiment 1 of the present invention applied to a lesion;
Fig. 19 is a schematic view of step two of an ablation assembly of embodiment 1 of the present invention applied to a lesion;
Fig. 20 is a schematic view of step three of an ablation assembly of embodiment 1 of the present invention applied to a lesion;
Fig. 21 is a schematic view of step four of an ablation assembly of embodiment 1 of the present invention applied to a lesion;
Fig. 22 is a schematic view of step five of an ablation assembly of embodiment 1 of the present invention applied to a lesion;
Fig. 23 is a schematic cross-sectional view of a place where an ablation needle mates with an insulated coaxial outer tube in accordance with embodiment 1 of the present invention;
Fig. 24 is a schematic cross-sectional view of a place where an ablation needle mates with an insulated coaxial outer tube in the prior art;

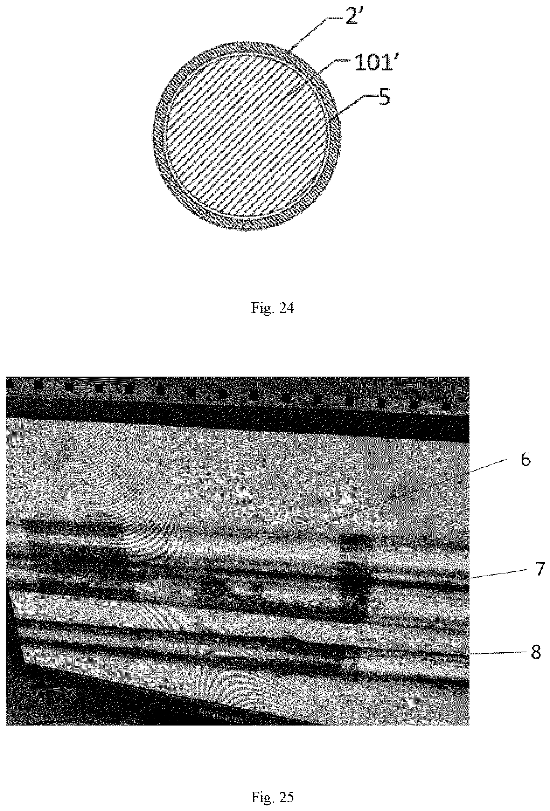

Fig. 25 shows the film burnout conditions after ablation using ablation needles with PI and PET insulating films in the prior art and an ablation needle with a parylene nanocoating as the insulating layer according to embodiment 1 of the present invention, wherein No. 6 in the figure represents the burnt condition of the insulated film of the insulated coaxial outer tube as a parylene nanocoating in the present invention, 7 represents the burnt condition of the prior art insulated coaxial outer tube using a PI film directly, and 8 represents the burnt condition of the prior art insulated coaxial outer tube using a PET film directly; and

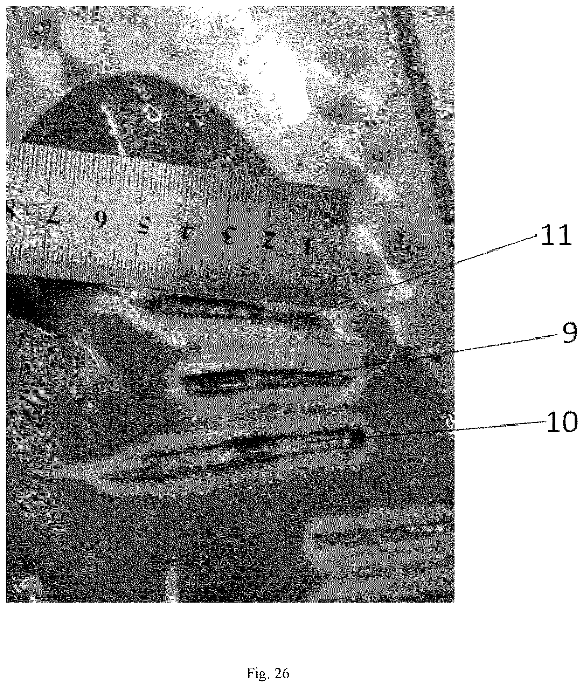

Fig. 26 shows the in vitro ablations of the liver using PI and PET insulating film ablation needles in the prior art, and the ablation needle with the insulating layer using a parylene nanocoating according to embodiment 1 of the present invention, wherein 9 represents the in vitro liver ablation of the ablation needle in the present invention having an insulated coaxial outer tube with a parylene nanocoating as the insulating layer, 10 represents the in vitro liver ablation result of the ablation needle in the prior art having an insulated coaxial outer tube directly using a PI film, and 11 represents the in vitro liver ablation result of the ablation needle in the prior art having an insulated coaxial outer tube directly using a PET film.

Reference numerals: 1-ablation needle; 101-ablation needle body; 102-ablation handle; 103-drainage tube; 104-thermocouple; 105-cable; 106-coaxial limiting member; 107-cooling medium inlet channel; 108-cooling medium outlet channel; 109-first cavity; 120-second cavity; 121-first cooling medium flow path; 122-second cooling medium flow path; 123-threading hole; 124-cooling cavity; 125-cooling medium inlet tube; 126-cooling medium outlet tube;
10201-rotating shaft; 10202-rear needle hub; 10203-adjustable rotary cap; 10204-cavity; 10205-fixed seat; 102051-ablation needle body fixed seat; 102052-drainage tube fixed seat; 10206-emboss; 10207-groove; 10208-first limiting unit; 10209-second limiting unit; 10210-snap ring; 10211-extending shaft;
2-insulated coaxial outer tube; 201-front needle hub; 202-coaxial outer tube; 203-insulating layer; 204-cutting edge; 3-biopsy needle; 4-puncture needle;
2'-insulated coaxial outer tube in the prior art; 101'-ablation needle body in the prior art; and 5-gap between the ablation needle body and the insulated coaxial outer tube in the prior art.

### Detailed Description of the Invention

An ablation needle assembly and an ablation system according to the present invention will now be described in further detail with reference to the accompanying drawings and specific embodiments. The advantages and features of the present invention will become more apparent in light of the following description.

Obviously, the described examples are only a part of the examples of the present invention, rather than all the examples. The assembly of the embodiments of the present invention, which are generally described and illustrated in the drawings herein, may be arranged and designed in a variety of different configurations.

Therefore, the following detailed description of the embodiments of the present invention provided in the accompanying drawings is not intended to limit the scope of the invention as claimed, but is merely representative of selected embodiments of the invention. Based on the embodiments of the present invention, all other embodiments obtained by one of ordinary skill in the art without involving any inventive efforts are within the scope of the present invention.

It should be noted that: like numbers refer to like items in the following figures, and therefore, once a certain item is defined in one figure, it need not be further defined and explained in the following figures.

In describing the present invention, it needs to be noted that the terms "first", "second", "third", and the like are used for descriptive purposes only and are not to be construed as indicating or implying relative importance.

Further, in the description of the present application, "proximal end", and "proximal end" of "distal end" are customary terms used in the medical field. Specifically, the "proximal end" refers to the end close to the operator, the "proximal end face" refers to the end face close to the operator, the "distal end" refers to the end away from the operator, and the "distal end face" refers to the end face away from the operator.

### Embodiment 1

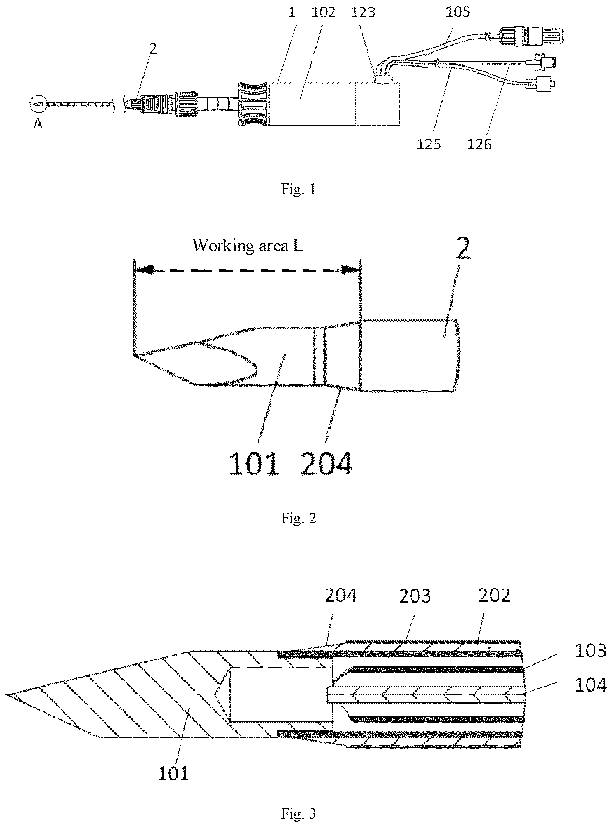

With reference to Figs. 1 to 3, an ablation needle assembly is provided for performing ablation operations, including a hollow insulated coaxial outer tube 2 and an ablation needle 1. The ablation needle 1 includes an ablation needle body 101 and an ablation handle 102 connected to the proximal end of the ablation needle body 101. The proximal portion of the ablation needle body 101 is fixed within the ablation handle 102. The ablation needle body 101 can be electrically connected to a radio frequency generator or other high-frequency energy generating device for ablation operations. Specifically, the proximal end of the ablation needle body 101 may be electrically connected to an energy generating device via a cable 105. The cable 105 passes through the threading hole 123 of the ablation handle 102 and extends out of the ablation handle 102. After the ablation needle body 101 is electrically connected to the radio frequency generator, the ablation needle body 101 transmits high-frequency current to the electrode working area L and then into the lesion tissue, causing ions in the tissue surrounding the electrode working area L to undergo high-speed oscillatory motion under the influence of current, generating frictional heat. This can raise the local temperature to 70°C to 100°C, so that direct heat is applied to lesion tissue or cells to induce coagulative denaturation and death. Damage to vessel walls surrounding the lesion cells leads to thrombus formation and interruption of blood supply, resulting in ischemic degeneration and necrosis of the lesion tissue. Additionally, estrogen/progesterone receptors in the tissues surrounding the tumor may be denatured and inactivated, inhibiting the growth, and thus achieving radio frequency ablation.

The ablation needle body 101 movably passes through the insulated coaxial outer tube 2, i.e., the ablation needle body 101 can freely move within the insulated coaxial outer tube 2. The proximal end of the insulated coaxial outer tube 2 is detachably connected to the distal end of the ablation handle 102. For example, the insulated coaxial outer tube 2 is detachably connected to the ablation handle 102 via a threaded connection, snap-fit connection, or other detachable connection methods. Considering sealing performance, a Luer connection is preferred, and a Luer connector with threads is particularly preferred with regard to the Luer connection due to better sealing and higher connection strength.

The insulated coaxial outer tube 2 is designed to be detachably connected to the ablation handle 102, so that the ablation needle 1 can be easily detached from the insulated coaxial outer tube 2. For the lesion tissue requiring multiple needle insertions, after the first needle insertion, the insulated coaxial outer tube 2 is still left in the tissue, providing a channel and positioning for other operations such as biopsy, avoiding repeated puncture, reducing the damage to the tissue, making ablation and other operations more convenient and efficient, simplifying the surgical process, and reducing the surgical time.

The ablation needle body 101 is closely fitted with the insulated coaxial outer tube 2, and the portion of the distal end of the ablation needle body 101 protruding out of the insulated coaxial outer tube 2 and the portion of the distal end of the insulated coaxial outer tube 2 without insulation form a single electrode.

The insulated coaxial outer tube 2 includes a coaxial outer tube 202, an insulating layer 203 wrapped around the coaxial outer tube 202, and a front needle hub 201 connected to the proximal end of the coaxial outer tube 202. The front needle hub 201 is detachably connected to the distal end of the ablation handle 102. The coaxial outer tube 202 is sleeved over the portion of the ablation needle body 101 exposed outside the ablation handle 102, and the ablation needle body 101 extends out from the distal end of the coaxial outer tube 202, forming the electrode working area L. The coaxial outer tube 202 has a portion at the distal end not covered by the insulating layer 203. The ablation needle body 101 is tightly connected with the coaxial outer tube 202, enabling the electrode working area L to be closely fitted with the portion of the distal end of the coaxial outer tube 202 that is not covered by the insulating layer 203, thereby forming a single electrode. That is, the electrode working area L serves as the working area of the single electrode. The formation of the single electrode ensures that no interfering radio frequency current is generated in normal tissue areas outside the target ablation area, thus avoiding ablation damage to normal tissues.

When the edge, covered by the insulating layer 203, in the distal end of the coaxial outer tube 202 transitions to the ablation needle body 101 protruding out of the insulated coaxial outer tube 2, a transition surface of the cutting edge 204 is formed. The cutting edge 204 and the coaxial outer tube 202 are not covered by the insulating layer 203, and the arrangement of the cutting edge 204 can effectively reduce the puncture force when the ablation needle body 101 puncture is performed.

The outer surface of the insulated coaxial outer tube 2 is covered with insulation, and serves as an insulating tube of the ablation needle body 101 during ablation, so that the ablation operation is performed on the portion of the ablation needle body 101 protruding out of the insulated coaxial outer tube 2. In addition to the above-mentioned implementation mode, the insulated coaxial outer tube 2 can also be directly made of an insulating material, such as a plastic tube such as PEEK, PI or PA which can meet the hardness requirements, and further, such as a ceramic tube such as high-alumina porcelain, talc porcelain or boron nitride. A non-insulating material is used to make the distal end of the insulated coaxial outer tube 2 such that the distal end forms a single electrode by being closely fitted with the ablation needle body 101 protruding out of the insulated coaxial outer tube 2.

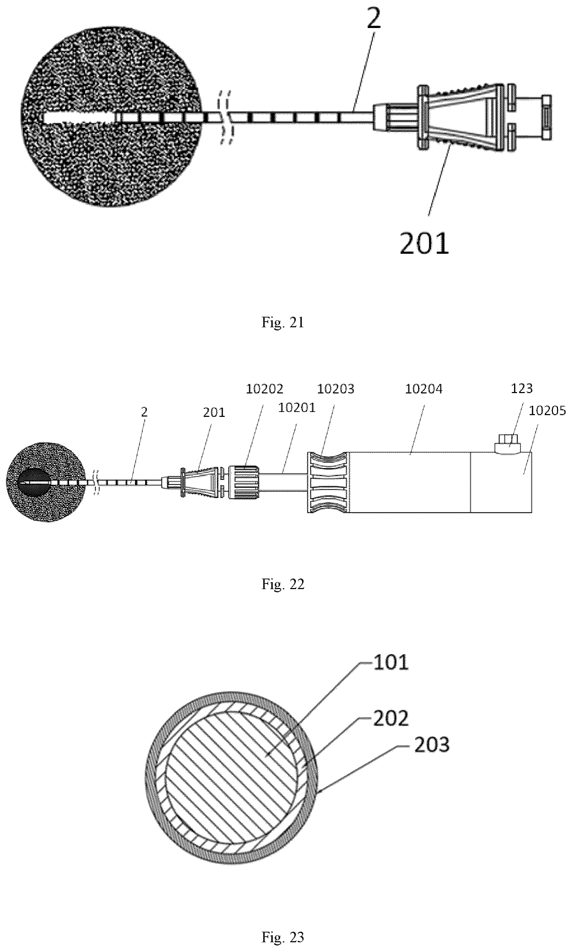

Preferably, as shown in Fig.23, the insulated coaxial outer tube 2 includes a coaxial outer tube 202 made of metallic material and an insulating layer 203 coated on the outer surface of the coaxial outer tube 202. The metallic material includes but is not limited to 304 stainless steel, 321 stainless steel, or 631 stainless steel. The insulating layer 203 includes but is not limited to PTFE coating, titanium nitride coating, parylene coating, etc. The insulating layer 203 is preferably a parylene nanocoating, which allows for a more intimate bond between the insulating layer 203 and the coaxial outer tube 202, enabling precise ablation range. The insulating layer 203 can form a dense protective film on the outer surface of the coaxial outer tube 202 via vacuum coating technology. The stainless steel material of the coaxial outer tube 202 shall have sufficient hardness to penetrate human tissues, and shall have excellent biocompatibility. The insulating coating shall have reliable insulation property, excellent biocompatibility and a small friction coefficient, and it is required that the insulating layer 203 shall be tightly bonded to the outer surface of the tube body of coaxial outer tube 202, so that the insulating layer 203 is not easy to fall off.

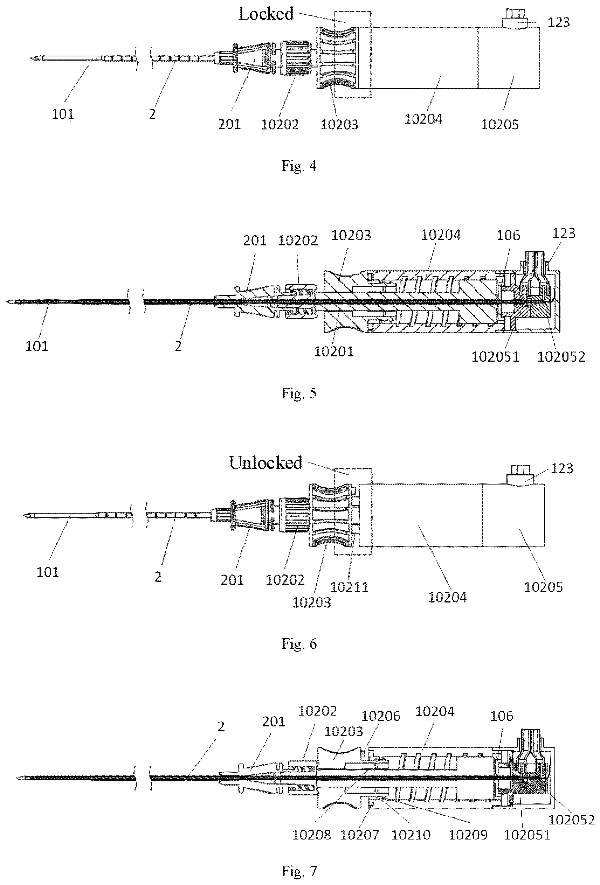

It needs to be noted that the ablation needle 1 can ablate pathological tissues at different sites and can also ablate nodules or tumors, and the sizes of different pathological tissues are different. In order to accommodate the ablation of different sizes of pathological tissues, in a preferred implementation mode of embodiment 1, with particular reference to Figs. 4 to 13, the ablation handle 102 is an ablation handle 102 with an adjustable axial length, the axial length of the ablation handle 102 is adjusted, and thus the distance between the insulated coaxial outer tube 2 and the proximal end of the ablation handle 102 is adjusted, and the ablation needle body 101 is kept stationary, so as to achieve real-time adjustment of the length of the distal end of the ablation needle body 101 extending out of the insulated coaxial outer tube 2.

Through the above-described implementation mode, during the adjustment of the axial length of the ablation handle 102, the distance between the insulated coaxial outer tube 2 and the proximal end of the ablation handle 102-i.e., the distance between the front needle hub 201 and the proximal end of the ablation handle 102-is adjusted. During this process, since the length of the insulated coaxial outer tube 2 remains unchanged and the ablation needle body 101 does not move, the insulated coaxial outer tube 2 moves relative to the ablation needle body 101. Therefore, the length by which the distal end of the ablation needle body 101 extends out of the insulated coaxial outer tube 2-i.e., the length of the working area L of the ablation needle 1-can be reversely adjusted, allowing the ablation needle 1 to adapt to the ablation of lesions of different sizes, and making it more flexible and effective for treating lesions of varying sizes. Moreover, during the adjustment of the axial length of the ablation handle 102, the ablation needle body 101 remains stationary-i.e., the ablation needle body 101 does not need to move-thereby not only avoiding the risk of leakage in the internal cooling medium circulation channel of the ablation needle 1, but also enabling clinicians to anticipate the operational space range and preventing insufficient operational space during surgeries by clinicians.

Additionally, it needs to be noted that, as shown in Fig. 24, in the ablation needle assembly currently used clinically, the insulated coaxial outer tube 2' external to the ablation needle body 101' is directly made of an insulating material, typically an ultra-thin plastic tubular film, while the ablation needle body 101' is typically a rigid metal tube, and a significant gap 5 exists between the insulated coaxial outer tube 2' and the ablation needle body 101'. Due to the presence of the gap 5, bodily fluids, blood, etc. may enter the gap 5 during clinical use of the ablation needle. On one hand, bodily fluid, blood, and the like entering the gap 5 may become dried, and the dried blood may adhere the insulated coaxial outer tube 2' to the ablation needle body 101', making it impossible for the user to adjust the position of the insulated coaxial outer tube 2' during clinical procedures to regulate the length of the electrode working area. If a greater force is applied for adjustment, the insulated coaxial outer tube 2' may fracture or shrink, resulting in failure of the electrode working area length adjustment function. On the other hand, when bodily fluid or blood enters the gap 5, a high-voltage electricity spark may be generated at the junction between the distal end edge of the insulated coaxial outer tube 2' and the electrode working area of the ablation needle body 101'. The high-voltage electricity spark can penetrate through and burn the insulated coaxial outer tube 2' at the distal end edge, causing film burnout. This leads to insulation failure at the distal end edge and consequently produces an ablation effect. This area is typically referred to as the insulation failure area. Over time, as the high-voltage electricity spark continues to occur, the insulation failure area gradually extends along the bodily fluid, blood, and the like within the gap 5 toward the ablation handle end of the ablation needle body 101', causing the ablation area to continuously expand. Ultimately, the actual electrode working area becomes significantly larger than the intended ablation size required by the physician, posing a considerable risk to clinical safety. As shown in Figs. 25 and 26, Fig. 25 schematically illustrates the film burnout condition of the insulated coaxial outer tube 2' of a prior-art ablation needle using PI and PET insulating films, respectively, during ablation of an in vitro liver, where obvious and severe film burnout can be observed. Fig. 26 schematically illustrates the in vitro liver ablation results, in which the preset working area length of the ablation needle is 4cm. It is clearly observed that, with the conventional insulated coaxial outer tube, precise adjustment of the electrode working area cannot be achieved, and all of them exceed the preset working area length.

In this embodiment, the insulated coaxial outer tube 2 preferably includes a coaxial outer tube 202 made of a metallic material and an insulating layer 203 coated on the outer surface of the coaxial outer tube 202 (as shown in Fig.23). Since both the ablation needle body 101 and the coaxial outer tube 202 are rigid metallic tubes, a relatively tight coaxial fit between the ablation needle body 101 and the coaxial outer tube 202 can be achieved through tolerance control. Even under such a tight fit, the ablation needle body 101 and the coaxial outer tube 202 can still maintain relative movability, allowing the ablation handle 102 to be controlled to adjust the electrode working area length L of the ablation needle body 101. Therefore, in this embodiment, the coaxial fit between the ablation needle body 101 and the insulated coaxial outer tube 2 is very tight, and the gap between the two is significantly smaller than that in a conventional ablation needle body and insulated coaxial outer tube. As a result, the entry of bodily fluids, blood, and the like into the gap between the ablation needle body 101 and the coaxial outer tube 202 is prevented or greatly reduced, thereby significantly reducing the adhesion force caused by the drying of the blood. Moreover, the insulated coaxial outer tube 2 is rigid and detachable from the ablation needle body 101, avoiding breakage or shrinking of the insulated coaxial outer tube 2 when the ablation handle 102 is adjusted.

Further, since the ablation size is closely related to the working area length of the ablation needle, the more precise the control of the ablation size, the higher the safety in clinical use. Due to the structure design of the existing insulated coaxial outer tube, there is a large gap between the insulated coaxial outer tube and the ablation needle body, resulting in inaccurate ablation size control and low clinical safety. In this embodiment, the coaxial outer tube 202 of the insulated coaxial outer tube 2 is externally covered by an insulating film 203, which is typically deposited or coated onto the outer surface of the coaxial outer tube 202 through a coating process. The insulating film 203 has strong adhesion to the coaxial outer tube 202, thus ensuring a tight fit between the two so as to prevent bodily fluids, blood, and the like from entering the space between the insulating film 203 and the coaxial outer tube 202. As a result, the high-voltage electricity spark film burnout does not occur at the distal end edge of the insulating film 203, and the insulating film 203 remains undamaged. Furthermore, even if a high-voltage electricity spark occurs at the distal end edge of the insulating film 203, the burn resistance of the coated insulating film is superior to that of the ultra-thin plastic tubular insulating film used in conventional insulated coaxial outer tubes. Therefore, the ablation size consistently matches the size of the electrode working area length adjusted by the physician, enabling precise control of the ablation size and significantly improving safety in clinical applications. As shown in Figs. 25 and 26, Fig. 25 illustrates the film burnout conditions of the insulated coaxial outer tube 2' of the prior-art ablation needles using PI and PET insulating films, respectively, during in vitro liver ablation, as well as the film burnout condition of the insulating film of the insulating film 203 of the insulated coaxial outer tube 2 of the present invention using a parylene nanocoating during in vitro liver ablation of the ablation needle. As clearly shown in Fig. 25, the insulation failure area of ablation needle No. 6 of the present invention is smaller than that of the conventional ablation needle No. 7 using PI insulating film directly, and also smaller than that of the conventional ablation needle No. 8 using PET film directly, indicating that the insulated coaxial outer tube structure of the present invention rarely experiences film burnout. Fig. 26 illustrates the in vitro liver ablations of conventional ablation needles with insulated coaxial outer tubes 2' using PI and PET insulating films, respectively, and the ablation needle of this embodiment with the insulating layer 203 made of a parylene nanocoating, with the ablation needles under a preset working area length of 4cm. Wherein 9 represents the in vitro liver ablation of the ablation needle in the present invention having an insulated coaxial outer tube with a parylene nanocoating as the insulating layer, 10 represents the in vitro liver ablation result of the ablation needle in the prior art having an insulated coaxial outer tube directly using a PI film, and 11 represents the in vitro liver ablation result of the ablation needle in the prior art having an insulated coaxial outer tube directly using a PET film. As clearly shown in Fig. 26, the ablation size of the present invention is more precise compared to those using PI insulating film and PET insulating film.

By axial movement of a rotating shaft 10201, the axial length of the ablation handle 102 is changed, thereby changing the distance between the proximal end of the insulated coaxial outer tube 2 and the proximal end of the ablation handle 102. The ablation handle 102 further includes the rotating shaft 10201, the ablation needle body 101 passes through the rotating shaft 10201 and is connected to the rotating shaft 10201 via a coaxially movable connection, the rotating shaft 10201 is coaxially arranged with the ablation handle 102, and the distal end of the rotating shaft 10201 is detachably connected to the proximal end of the insulated coaxial outer tube 2, that is, the distal end of the rotating shaft 10201 is detachably connected to the front needle hub 201 of the insulated coaxial outer tube 2;

The rotating shaft 10201 moves axially to change the axial length of the ablation handle 102, thereby changing the distance between the proximal end of the insulated coaxial outer tube 2 and the proximal end of the ablation handle 102. Further, the ablation handle 102 includes a rear needle hub 10202 and a hand-held portion, wherein the rear needle hub 10202 is detachably connected to the front needle hub 201 of the insulated coaxial outer tube 2, the distal end of the rotating shaft 10201 is fixedly connected to the rear needle hub 10202, and the proximal end of the rotating shaft 10201 is connected to the hand-held portion.

By adjusting the distance between the distal end of the hand-held portion and the rear needle hub 10202, the axial length of the ablation handle 102 is adjusted, thereby adjusting the distance between the front needle hub 201 and the hand-held portion, thus adjusting the electrode working area L length of the ablation needle body 101 protruding out of the insulated coaxial outer tube 2. Providing the rear needle hub 10202 between the rotating shaft 10201 and the insulated coaxial outer tube 2 can solve the connection issue between the rotating shaft 10201 and the proximal end of the insulated coaxial outer tube 2.

In one preferred implementation mode, rotation of the rotating shaft 10201 is employed to achieve axial movement of the rotating shaft 10201; the rotating shaft 10201 is coaxially provided within the hand-held portion and threadedly connected thereto, and the distal end of the rotating shaft 10201 is fixedly connected to the rear needle hub 10202; and by rotating and adjusting the rotating shaft 10201, the rotating shaft 10201 moves axially to adjust the distance between the rear needle hub 10202 and the distal end of the hand-held portion, thereby adjusting the length of the ablation handle 102.

In embodiment 1, in one preferred implementation mode, the hand-held portion includes an adjustable rotary cap 10203 provided at the distal end, a cavity 10204, and a fixed seat 10205 fixedly connected to the cavity 10204; the proximal end of the ablation needle body 101 is fixedly connected to the fixed seat 10205; preferably, a coaxial limiting member 106 is provided on the distal end surface of the fixed seat 10205, the coaxial limiting member 106 being coaxially arranged with the ablation needle body 101 and fixedly connected to the fixed seat 10205; and the provision of the coaxial limiting member 106 increases the coaxial limiting length at the proximal end of the ablation needle body 101, thereby achieving better coaxiality between the ablation needle body 101 and the fixed seat 10205.

The proximal end of the rotating shaft 10201 is threadedly connected to the interior of the cavity 10204, i.e., the proximal end of the rotating shaft 10201 is provided with external threads, and the inner wall of the cavity 10204 is provided with internal threads at the position cooperating with the proximal end of the rotating shaft 10201, the external threads being engaged with the internal threads to achieve threaded connection.

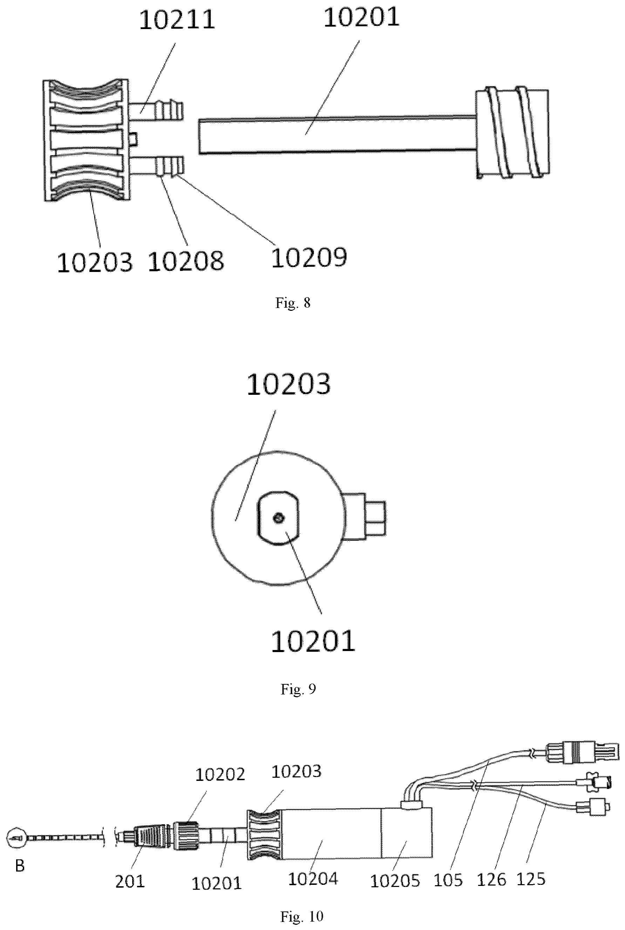

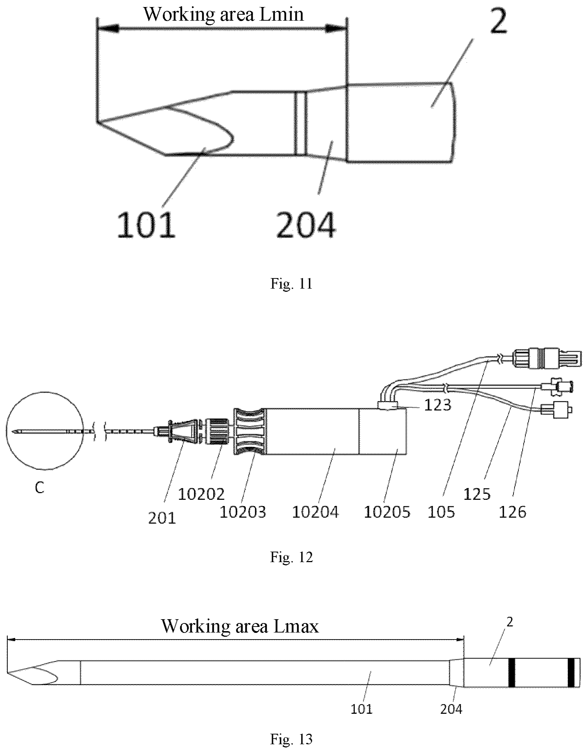

Rotating the adjustable rotary cap 10203 causes the rotating shaft 10201 and the adjustable rotary cap 10203 to rotate synchronously, thereby adjusting the rotating shaft 10201 to move axially. The adjustable rotary cap 10203 rotates to cause the rotating shaft 10201 to rotate synchronously with the adjustable rotary cap 10203. Since the rotating shaft 10201 is threadedly connected to the cavity 10204 and the cavity 10204 remains stationary, the rotating shaft 10201 moves axially. The rotating shaft 10201 drives the rear needle hub 10202 to move, thereby adjusting the axial length of the ablation handle 102. The ablation needle body 101 is fixedly connected to the fixed seat 10205 and is coaxially movably connected to the rotating shaft 10201. When the rotating shaft 10201 undergoes axial displacement due to the rotation of the adjustable rotary cap 10203, the axial length of the ablation handle 102 changes, thereby causing an inversely proportional change in the axial length of the ablation needle body 101 extending out of the hand-held portion. Since the length of the insulated coaxial outer tube 2 remains constant, rotating the adjustable rotary cap 10203 results in a change in the length of the ablation needle body 101 extending out of the hand-held portion, equivalent to a change in the length of the electrode working area L of at the distal end of the ablation needle body 101 extending out of the cutting edge 204 of the insulated coaxial outer tube 2, thereby enabling adjustment of the length of the electrode working area L of the ablation needle 1. In the present embodiment, the adjustable range of the electrode working area L of the ablation needle 1 is 5mm to 235mm. Specifically, Figs. 10 to 13 illustrate the shortest state and longest state of the electrode working area L of the ablation needle 1. The shortest length of the electrode working area L is denoted as Lmin (Figs. 10 and 11), at which time the ablation handle 102 is at its maximum length; and the longest length of the electrode working area L is denoted as Lmax (Figs. 12 and 13), at which time the ablation handle 102 is at its minimum length. To facilitate the understanding of the length change, scale markings are provided on the non-threaded portion of the rotating shaft 10201 in this embodiment such that the current length of the electrode working area L can be known. It can be seen that the present embodiment enables smooth adjustment of the electrode working area within the range of Lmin to Lmax, thereby achieving smooth adjustment of the electrode working area length of the ablation needle body within a certain range, i.e., stepless adjustment.

To prevent accidental touching of the adjustable rotary cap 10203 during ablation by the ablation needle 1, which could inadvertently adjust the electrode working area L of the ablation needle body 101, a locking structure is provided at the connection between the adjustable rotary cap 10203 and the distal end of the cavity 10204. Specifically, as shown in Figs. 4 to 9, after the adjustable rotary cap 10203 is connected to the cavity 10204, the locking structure locks to lock the rotation of the adjustable rotary cap 10203, thereby preventing the adjustment of the axial length of the ablation handle 102. When the locking structure is in the unlocked state, the axial length of the ablation handle 102 can be adjusted. By adopting the above implementation mode, risks caused by accidental touching of the adjustable rotary cap 10203 leading to changes in the working area length of the ablation needle 1 can be prevented.

In one of the preferred implementation modes, the adjustable rotary cap 10203 and the cavity 10204 may be locked via a snap-fit connection. The locking structure includes an emboss 10206 and a groove 10207. A plurality of embosses 10206 are provided on the proximal end surface of the adjustable rotary cap 10203, and a groove 10207 is formed on the distal end surface of the cavity 10204. Alternatively, a plurality of grooves 10207 are formed on the proximal end surface of the adjustable rotary cap 10203, and an emboss 10206 is provided on the distal end surface of the cavity 10204.

The emboss 10206 is embedded within the groove 10207 to restrict the rotation of the adjustable rotary cap 10203.

Furthermore, in order to prevent unintended unlocking of the locking structure during ablation with ablation needle 1, axial movement of adjustable rotary cap 10203 is further restricted based on the groove 10207 and emboss 10206, thereby further locking the adjustable rotary cap 10203. The proximal end surface of the adjustable rotary cap 10203 has a plurality of extending shafts 10211 extending toward cavity 10204. A first limiting unit 10208 is provided on the extending shaft 10211. A snap ring 10210 is arranged circumferentially inside cavity 10204. When the adjustable rotary cap 10203 is connected to the cavity 10204, the first limiting unit 10208 snaps into the side of the snap ring 10210 close to the proximal end, thereby restricting axial movement of the adjustable rotary cap 10203. The above implementation mode can prevent unintended unlocking of the adjustable rotary cap 10203.

To limit the axial movement of the adjustable rotary cap 10203 to remain still during the movement of the rotating shaft 10201, the extending shaft 10211 is further provided with a second limiting unit 10209. When the adjustable rotary cap 10203 is separated from the cavity 10204, the snap ring 10210 is embedded between the first limiting unit 10208 and the second limiting unit 10209, thereby restricting axial movement of the adjustable rotary cap 10203 during movement of the rotating shaft 10201. In order to make it easy for the first limiting unit 10208 to cross over the snap ring 10210 when the rotary cap is unlocked and pulled out, the first limiting unit 10208 is preferably provided as a circular arc protrusion as shown in Figs. 7 and 8.

In the above implementation mode, the ablation handle 102 is a handle equipped with a locking structure and capable of adjusting axial length. Specifically, the adjustable rotary cap 10203 and the cavity 10204 have a locked state and an unlocked state. In the locked state, the length of the electrode working area L of the ablation needle 1 cannot be adjusted; and in the unlocked state, the length of the electrode working area L of the ablation needle 1 can be adjusted by rotating the adjustable rotary cap 10203.

Refer again to Figs. 4 and 5, in the locked state of the locking structure, the emboss 10206 on the adjustable rotary cap 10203 is embedded into the groove 10207 on the cavity 10204, preventing rotation of the adjustable rotary cap 10203. Meanwhile, the first limiting unit 10208 on the rotary cap is pressed into the side of the snap ring 10210 on the cavity 10204 close to the proximal end, preventing movement of the adjustable rotary cap 10203, thereby locking the adjustable rotary cap 10203 and fixing the length of the electrode working area L of the ablation needle 1.

When it is necessary to change the length of the electrode working area L of the ablation needle 1, the locking structure needs to be unlocked. Referring to Figs. 5 and 6, at this time, the adjustable rotary cap 10203 is pulled toward one side at the distal end, allowing the first limiting unit 10208 on the rotary cap to cross over the snap ring 10210 of the cavity 10204, so that the snap ring 10210 is embedded between the first limiting unit 10208 and the second limiting unit 10209. At this time, the emboss 10206 disengages from the groove 10207, allowing relative rotation between the adjustable rotary cap 10203 and the cavity 10204. Rotating the adjustable rotary cap 10203 drives the rotating shaft 10201 to rotate synchronously in the same direction. Since the rotating shaft 10201 is threadedly connected to the cavity 10204, the rotating shaft 10201 drives the rear needle hub 10202 to perform axial movement, thereby changing the length of the electrode working area L of the ablation needle 1.

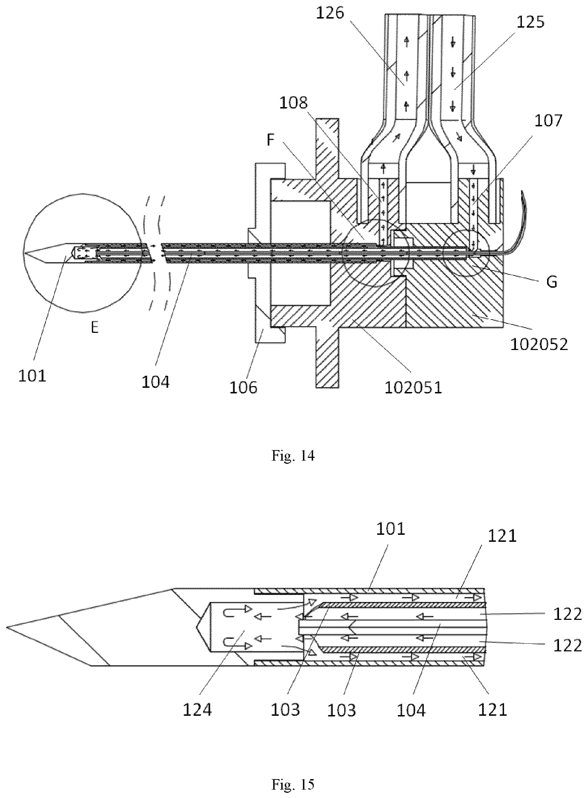

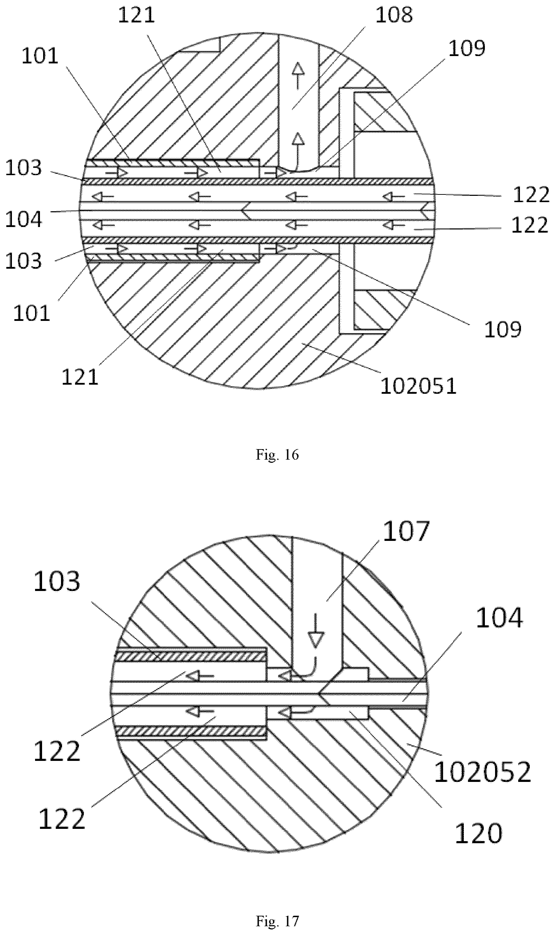

Refer to Figs. 14 to 17, the portion of the electrode working area L of the ablation needle 1 in contact with the tissue delivers radio frequency energy, causing the tissue to heat up and undergo coagulative necrosis to achieve a therapeutic effect. However, excessive local temperature may damage surrounding normal tissue not intended for ablation. Therefore, except for the solid needle tip part, the ablation needle body 101 of the ablation needle 1 is constructed with a hollow inner cavity in other parts, and the inner cavity is configured to deliver a gaseous or liquid cooling medium (e.g., cooling water) to lower the temperature and control the temperature during ablation operation. Generally, the channel for delivering the cooling medium to the inner cavity of the ablation needle 1 is arranged parallel to the axis of the ablation needle 1.

In some implementation modes of this embodiment, the fixed seat 10205 is provided therein with a cooling medium inlet channel 107 and a cooling medium outlet channel 108. The cooling medium inlet channel 107 forms a cooling medium inlet on the side surface of the fixed seat 10205, and the cooling medium outlet channel 108 forms a cooling medium outlet on the side surface of the fixed seat 10205. The side surface of the fixed seat 10205 refers to the side surface excluding the proximal end surface of the fixed seat 10205.

The straight line in which the cooling medium inlet channel 107 is located and the straight line in which the cooling medium outlet channel 108 is located respectively intersect with the axis of the ablation needle 1, the straight line in which the cooling medium inlet channel 107 is located refers to the extension line of the cooling medium inlet channel 107 or a line parallel to the cooling medium inlet channel 107, and the straight line in which the cooling medium outlet channel 108 is located refers to the extension line of the cooling medium outlet channel 108 or a line parallel to the cooling medium outlet channel 108.

In the above-mentioned implementation mode, the cooling medium inlet channel 107 and the cooling medium outlet channel 108 communicating with an external cooling device are arranged to intersect with the axis of the ablation needle 1, and the cooling medium inlet and the cooling medium outlet are located on the side surface of the ablation handle 102 other than the proximal end surface, which greatly reduces the length of the ablation handle 102 with respect to the existing technical solution that the cooling medium inlet channel 107 and the cooling medium outlet channel 108 are parallel to the axis of the ablation needle 1.

It needs to be noted that the ablation needle assembly is used under a medical imaging device, and clinical operators often face limited operational space, especially in annular CT environments. By adopting the solution of this embodiment, the size of the ablation handle 102 can be reduced, thereby providing clinicians with increased operational space, which has significant clinical implications.

It is further preferred that the cooling medium inlet channel 107 is in a straight line perpendicular to the axis of the ablation needle 1; and/or
the straight line where the cooling medium outlet channel 108 is located is perpendicular to the axis of the ablation needle 1.

It is further preferred that the straight lines where the cooling medium inlet channel 107 and the cooling medium outlet channel 108 are located are both perpendicular to the axis of the ablation needle 1. In the perpendicular state, the axial length of the ablation handle 102 can be minimized to the maximum extent, and it can be ensured that the cooling medium can smoothly flow into the channel in the ablation needle 1.

It is further preferred that the ablation needle 1 further comprises a drainage tube 103, the drainage tube 103 being coaxially arranged in the ablation needle body 101.

The fixed seat 10205 includes an ablation needle body fixed seat 102051 and a drainage tube fixed seat 102052, wherein the proximal end of the ablation needle body 101 is fixedly connected to the ablation needle body fixed seat 102051, and meanwhile, the ablation needle fixed seat 102051 seals the gap between the ablation needle body 101 and the ablation needle body fixed seat 102051, so that a first cavity 109 is formed at the gap where the proximal end of the ablation needle body 101 and the ablation needle body fixed seat 102051 cooperate.

The proximal end of the drainage tube 103 is fixedly connected to the drainage tube fixed seat 102052, and meanwhile, the drainage tube fixed seat 102052 seals the gap where the drainage tube 103 and the drainage tube fixed seat 102052 cooperate, thereby forming a second cavity 120 at the mating gap where the proximal end of the drainage tube 103 and the drainage tube fixed seat 102052 cooperate.

The first cavity 109 is in communication with the cooling medium outlet channel 108, the cooling medium outlet channel 108 being located within the ablation needle body fixed seat 102051; and the second cavity 120 is in communication with the cooling medium inlet channel 107, the cooling medium inlet channel 107 being located within the drainage tube fixed seat 102052.

The drainage tube 103 is provided therein with a second inner cavity extending from the proximal end to the distal end, the second inner cavity forming a second cooling medium flow path 122, and the second cooling medium flow path 122 being in communication with the second cavity 120.

The ablation needle body 101 is provided therein with a first inner cavity extending from the proximal end to the distal end, and a gap between the first inner cavity and the drainage tube 103 forms the first cooling medium flow path 121, wherein the first cooling medium flow path 121 is connected to the first cavity 109.

The portion of the ablation needle body 101 extending out of the insulated coaxial outer tube 2 has in it a cooling cavity 124, the cooling cavity 124 being provided at the distal end of the drainage tube 103, and the cooling cavity 124 being in communication with both the first cooling medium flow path 121 and the second cooling medium flow path 122.

The circulation direction of the cooling medium within the ablation needle body 101 in this embodiment is as shown in Figs. 14 to 17. The cooling medium flows into the cooling medium inlet channel 107 through the cooling medium inlet tube 125, then into the second cavity 120 and the second cooling medium flow path 122, subsequently into the cooling cavity 124 to cool the electrode working area L, then into the first cooling medium flow path 121 and the first cavity 109, further into the cooling medium outlet channel 108, and finally out of the ablation needle 1 through the cooling medium outlet tube 126. And then the cooling medium flows in.

To detect in real time the temperature of the circulating cooling medium within the ablation needle body 101, a thermocouple 104 is provided within the drainage tube 103. The thermocouple 104 is arranged coaxially within the drainage tube 103, passes sequentially through the ablation needle body fixed seat 102051 and the drainage tube fixed seat 102052, and fixedly connects the two. The thermocouple 104 extends out from the proximal end of the drainage tube fixed seat 102052 and is led out through a threading hole 123 on the side surface of the drainage tube fixed seat 102052, where it is electrically connected to a cable 105, enabling real-time detection of the cooling medium temperature on the display screen of the energy generating device. The gap between the thermocouple 104 and the drainage tube 103 in the inner cavity constitutes the second cooling medium flow path 122.

In a specific implementation of this embodiment, as shown in Fig.14, the threading hole 123, the cooling medium inlet, and the cooling medium outlet are all provided on the side surface of the fixed seat 10205. The cooling medium outlet channel is communicated with the cooling device directly via the cooling medium outlet tube, and the cooling medium inlet channel is communicated with the cooling device directly via the cooling medium inlet tube.

In a preferred implementation mode of embodiment 1, the ablation needle assembly further includes a biopsy needle. The biopsy needle and the ablation needle 1 are alternately inserted into the insulated coaxial outer tube 2 and detachably connected to the insulated coaxial outer tube 2.

In a preferred implementation mode of embodiment 1, the ablation needle assembly further includes a puncture needle. The puncture needle and the ablation needle 1 are alternately inserted into the insulated coaxial outer tube 2 and detachably connected to the insulated coaxial outer tube 2, wherein the distal end of the puncture needle extends out of the insulated coaxial outer tube 2.

During conventional ablation using the ablation needle 1 of this embodiment, the ablation needle 1 movably passes through the insulated coaxial outer tube 2, and then the rear needle hub 10202 is connected to the Luer connector of the front needle hub 201 to combine into the ablation needle assembly. After the electrode working area L is adjusted to an appropriate length, the lesion site is punctured followed by ablation under medical imaging.

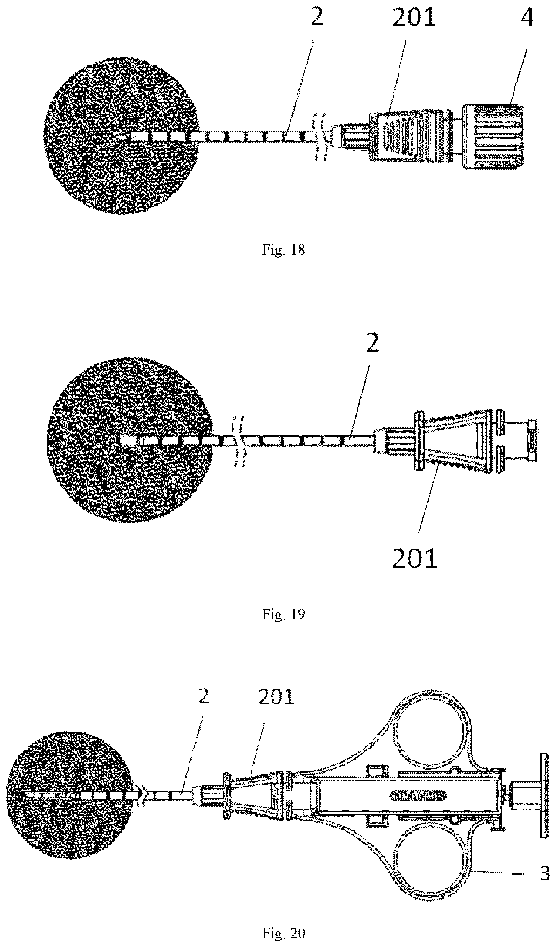

In addition to the above-mentioned conventional ablation, the ablation needle assembly of the present embodiment can also perform post-biopsy ablation, as specifically shown in figures 18 to 22. Step one, referring to figure 18, after the puncture needle 4 movably passes through the insulated coaxial outer tube 2 and is connected with a Luer connector, the puncture needle is punctured to a lesion position under medical imaging;
step two, referring to Fig. 19, the Luer connector of the front needle hub 201 is rotated, loosening the connection between the puncture needle 4 and the Luer connector of the insulated coaxial needle tube, and pulling out the puncture needle 4, in which process the insulated coaxial needle tube is kept stationary;
step three, referring to Fig. 20, the biopsy needle 3 movably passes through the insulated coaxial outer tube 2 and is connected via a Luer connector, and the biopsy needle 3 is fired under medical imaging to obtain a biopsy tissue sample;
step four, referring to Fig. 21, the Luer connector of the front needle hub 201 is rotated, loosening the connection between the biopsy needle 3 and the Luer connector of the insulated coaxial outer tube 2, and pulling out the biopsy needle 3, wherein the insulated coaxial outer tube 2 also remains stationary in the process; and
step five, referring to Fig. 22, the adjustable rotary cap 10203 of the ablation needle 1 is rotated to adjust the length of the electrode working area L to a suitable length; and after movably passing through the insulated coaxial outer tube 2, it is connected via a Luer connector and ablation is performed under medical imaging.

In this embodiment, the insulated coaxial outer tube 2 is detachably connected to the ablation needle **1,** the biopsy needle 3 or the puncture needle 4. In some surgical procedures, the needle needs to be inserted, such as the post-biopsy ablation as described above. After the first insertion of the needle, the insulated coaxial outer tube 2 is left in the tissue to provide a channel for subsequent operations, so that the biopsy or ablation needle 1 can reach the desired biopsy position, repeated puncture is avoided, and the damage to the tissue is reduced.

### Embodiment 2

An ablation system includes the ablation needle assembly of embodiment 1, and an energy generating device and a cooling device electrically connected to the ablation needle body of the ablation needle.

The energy generating device is a radio frequency generator or other high-frequency energy generators; and a cable connected to the proximal end of the ablation needle body is provided with an aviation connector. The aviation connector is inserted into the radio frequency generator or other high-frequency energy generators, i.e., the ablation needle body is electrically connected to the radio frequency generator or other high-frequency energy generators; and the cooling medium inlet and cooling medium outlet on the side surface of the ablation handle's proximal end, i.e., the side surface of the fixed seat 10205, are communicated with the cooling device correspondingly via a pipeline.

Implementation modes of the present invention have been described above in detail with reference to the accompanying drawings, but the present invention is not limited to the above-described implementation modes. Even if various changes are made to the present invention, if these changes fall within the scope of the claims and equivalent technologies of the present invention, they still fall within the scope of protection of the present invention.

## Claims

1. An ablation needle assembly, **characterized by** comprising an insulated coaxial outer tube and an ablation needle, wherein the ablation needle comprises an ablation needle body and an ablation handle connected to a proximal end of the ablation needle body, the ablation needle body movably passes through an interior of the insulated coaxial outer tube, and the insulated coaxial outer tube and the ablation handle are detachably connected; and
a portion of a distal end of the ablation needle body extending out of the insulated coaxial outer tube cooperates closely with an uninsulated portion of a distal end of the insulated coaxial outer tube to form a single electrode.

2. The ablation needle assembly according to claim 1, **characterized in that** the ablation handle has an adjustable axial length,
changing an axial length of the ablation handle while keeping the ablation needle body stationary, so as to enable real-time adjustment of a length by which the distal end of the ablation needle body extends out of the insulated coaxial outer tube, thereby achieving real-time adjustment of an electrode working area length change of the ablation needle body.

3. The ablation needle assembly according to claim 2, **characterized in that** the ablation handle comprises a rotating shaft, a rear needle hub, and a hand-held portion, wherein the rear needle hub is detachably connected to a proximal end of the insulated coaxial outer tube, a distal end of the rotating shaft is fixedly connected to the rear needle hub, and a proximal end of the rotating shaft is connected with the hand-held portion;
the ablation needle body passes through the rotating shaft and is coaxially and movably connected to the rotating shaft, and the rotating shaft is coaxially arranged with the ablation handle; and
the rotating shaft moves axially, and adjusting a distance between the rear needle hub and the hand-held portion is performed so as to achieve the axial length change of the ablation handle.

4. The ablation needle assembly according to claim 3, **characterized in that** the proximal end of the rotating shaft is connected with a thread of the hand-held portion; and
by rotating and adjusting the rotating shaft, the rotating shaft moves axially, thereby achieving a length of the rotating shaft exposed from the hand-held portion, and thus adjusting the distance between the rear needle hub and the hand-held portion so as to change the axial length of the ablation handle.

5. The ablation needle assembly according to claim 4, **characterized in that** the hand-held portion comprises an adjustable rotary cap provided at the distal end, a cavity, and a fixed seat fixedly connected to the cavity, and a proximal end of the rotating shaft is threaded with an interior of the cavity;
a locking structure is provided at a connection between the adjustable rotary cap and the cavity, and when the locking structure is in a locked state, a rotation of the adjustable rotary cap is locked; and when the locking structure is in an unlocked state, the electrode working area length of the ablation needle body can be adjusted; and
when the locking structure is in the unlocked state, rotating the adjustable rotary cap causes the rotating shaft to rotate synchronously with the adjustable rotary cap, thereby causing an axial movement of the rotating shaft.

6. The ablation needle assembly according to claim 5, **characterized in that** the locking structure comprises an emboss and a groove, and
a plurality of embosses are provided on a proximal end surface of the adjustable rotary cap, and a groove is formed on a distal end surface of the cavity; or
a plurality of grooves are formed on the proximal end surface of the adjustable rotary cap, and an emboss is provided on the distal end surface of the cavity; and
the emboss is embedded in the groove to lock the rotation of the adjustable rotary cap.

7. The ablation needle assembly according to claim 5, **characterized in that** the locking structure further comprises a first limiting unit and a snap ring, and
the proximal end surface of the adjustable rotary cap has a plurality of extending shafts extending toward the cavity, the first limiting unit is provided on the extending shaft, and an inner surface of the cavity is provided with the snap ring; and when the adjustable rotary cap is connected to the cavity, the first limiting unit snaps into one side of the snap ring close to the proximal end, thereby limiting axial movement of the adjustable rotary cap.

8. The ablation needle assembly according to claim 7, **characterized in that** the extending shaft is further provided with a second limiting unit, and when the adjustable rotary cap is separated from the cavity, the snap ring is embedded between the first limiting unit and the second limiting unit, thereby limiting axial movement of the adjustable rotary cap during a movement of the rotating shaft.

9. The ablation needle assembly according to claim 2, **characterized in that** insulated coaxial outer tube comprises a coaxial outer tube made of a metal material and an insulating layer coated on an outer surface of the coaxial outer tube.

10. The ablation needle assembly according to claim 1, **characterized in that** a cooling medium inlet channel and a cooling medium outlet channel are provided in the ablation handle, the cooling medium inlet channel forming a cooling medium inlet at a side surface of the ablation handle and the cooling medium outlet channel forming a cooling medium outlet at the side surface of the ablation handle;
a straight line on which the cooling medium inlet channel is located and a straight line on which the cooling medium outlet channel is located respectively intersect with an axis of the ablation needle; and
the side surface of the ablation handle refers to a side surface excluding a proximal end surface of the ablation handle.

11. The ablation needle assembly according to claim 10, **characterized in that** the ablation needle further comprises a drainage tube coaxially provided within the ablation needle body;
the ablation handle comprises an ablation needle body fixed seat and a drainage tube fixed seat, wherein a first cavity is formed at the gap where the proximal end of the ablation needle body and the ablation needle body fixed seat cooperate, and a second cavity is formed at the gap where a proximal end of the drainage tube and the drainage tube fixed seat cooperate; and
the first cavity is in communication with the cooling medium outlet channel, the cooling medium outlet channel being at least partially located within the ablation needle body fixed seat; and the second cavity is in communication with the cooling medium inlet channel, the cooling medium inlet channel being at least partially located within the drainage tube fixed seat.

12. The ablation needle assembly according to claim 11, **characterized in that** the drainage tube is provided therein with a second inner cavity extending from the proximal end to the distal end, the second inner cavity forming a second cooling medium flow path, and the second cooling medium flow path being in communication with the second cavity; and
the ablation needle body is provided therein with a first inner cavity extending from the proximal end to the distal end, and a gap between the first inner cavity and the drainage tube forms a first cooling medium flow path, the first cooling medium flow path being in communication with the first cavity.

13. The ablation needle assembly according to claim 12, **characterized in that** a portion of the ablation needle body extending out of the insulated coaxial outer tube has a cooling cavity inside, and the cooling cavity is in communication with both the first cooling medium flow path and the second cooling medium flow path.

14. The ablation needle assembly according to any one of claims 1 to 13, **characterized in that** the ablation needle assembly further comprises a biopsy needle and/or a puncture needle, the biopsy needle and the ablation needle are alternately inserted into the insulated coaxial outer tube and detachably connected to the insulated coaxial outer tube; and
the puncture needle and the ablation needle are alternately inserted within the insulated coaxial outer tube and detachably connected to the insulated coaxial outer tube, with a distal end of the puncture needle extending out of the insulated coaxial outer tube.

15. An ablation system, **characterized by** comprising the ablation needle assembly of any one of claims 1 to 14 and an energy generating device electrically connected to the ablation needle body of the ablation needle.

## Amended claims

### Amended claims under Art. 19.1 PCT

1. An ablation needle assembly, **characterized by** comprising an insulated coaxial outer tube and an ablation needle, wherein the ablation needle comprises an ablation needle body and an ablation handle connected to a proximal end of the ablation needle body, and the ablation needle body movably passes through an interior of the insulated coaxial outer tube;
a portion of a distal end of the ablation needle body extending out of the insulated coaxial outer tube cooperates closely with an uninsulated portion of a distal end of the insulated coaxial outer tube to form a single electrode;
the ablation handle comprises a rotating shaft, a rear needle hub, and a hand-held portion, wherein the rear needle hub is detachably connected to a proximal end of the insulated coaxial outer tube, and a distal end of the rotating shaft is fixedly connected to the rear needle hub;
the ablation needle body passes through the rotating shaft and is coaxially and movably connected to the rotating shaft, and the rotating shaft is coaxially arranged with the ablation handle;
the hand-held portion comprises an adjustable rotary cap provided at the distal end, a cavity, and a fixed seat fixedly connected to the cavity, and a proximal end of the rotating shaft is threaded with an interior of the cavity;
by rotating and adjusting the adjustable rotary cap, the rotating shaft moves axially, thereby adjusting a length of the rotating shaft exposed from the hand-held portion, and thus adjusting a distance between the rear needle hub and the hand-held portion, so as to change an axial length of the ablation handle while keeping the ablation needle body stationary, so as to enable real-time adjustment of a length by which the distal end of the ablation needle body extends out of the insulated coaxial outer tube, thereby achieving real-time adjustment of an electrode working area length change of the ablation needle body;
the insulated coaxial outer tube comprises a coaxial outer tube made of metallic material and an insulating layer coated on an outer surface of the coaxial outer tube; and
the ablation needle body is tightly fitted with the coaxial outer tube to prevent or reduce an ingress of bodily fluid or blood into a gap between the ablation needle body and the coaxial outer tube, thereby reducing an adhesion force caused by blood drying, and avoiding a fracture or shrinkage of the insulated coaxial outer tube, thus enabling stepless adjustment of the electrode working area of the ablation needle body; and the insulating layer is deposited on the outer surface of the coaxial outer tube by a coating process, so that the insulating layer is tightly fitted with the coaxial outer tube, preventing bodily fluid or blood from entering a space between the insulating layer and the coaxial outer tube, and avoiding high-voltage spark film burnout at a distal end edge of the insulating layer, thereby enabling precise adjustment of the length of the electrode working area of the ablation needle body and realizing clinical safety of the ablation needle.

2. The ablation needle assembly according to claim 1, **characterized in that**
a locking structure is provided at a connection between the adjustable rotary cap and the cavity, and when the locking structure is in a locked state, a rotation of the adjustable rotary cap is locked; and when the locking structure is in an unlocked state, the electrode working area length of the ablation needle body can be adjusted; and
when the locking structure is in the unlocked state, rotating the adjustable rotary cap causes the rotating shaft to rotate synchronously with the adjustable rotary cap, thereby causing an axial movement of the rotating shaft.

3. The ablation needle assembly according to claim 2, **characterized in that** the locking structure comprises an emboss and a groove, and
a plurality of embosses are provided on a proximal end surface of the adjustable rotary cap, and a groove is formed on a distal end surface of the cavity; or
a plurality of grooves are formed on the proximal end surface of the adjustable rotary cap, and an emboss is provided on the distal end surface of the cavity; and
the emboss is embedded in the groove to lock the rotation of the adjustable rotary cap.

4. The ablation needle assembly according to claim 2, **characterized in that** the locking structure further comprises a first limiting unit and a snap ring, and
the proximal end surface of the adjustable rotary cap has a plurality of extending shafts extending toward the cavity, the first limiting unit is provided on the extending shaft, and an inner surface of the cavity is provided with the snap ring; and when the adjustable rotary cap is connected to the cavity, the first limiting unit snaps into one side of the snap ring close to the proximal end, thereby limiting axial movement of the adjustable rotary cap.

5. The ablation needle assembly according to claim 4, **characterized in that** the extending shaft is further provided with a second limiting unit, and when the adjustable rotary cap is separated from the cavity, the snap ring is embedded between the first limiting unit and the second limiting unit, thereby limiting axial movement of the adjustable rotary cap during a movement of the rotating shaft.

6. The ablation needle assembly according to claim 1, **characterized in that** a cooling medium inlet channel and a cooling medium outlet channel are provided in the ablation handle, the cooling medium inlet channel forming a cooling medium inlet at a side surface of the ablation handle and the cooling medium outlet channel forming a cooling medium outlet at the side surface of the ablation handle;
a straight line on which the cooling medium inlet channel is located and a straight line on which the cooling medium outlet channel is located respectively intersect with an axis of the ablation needle; and
the side surface of the ablation handle refers to a side surface excluding a proximal end surface of the ablation handle.

7. The ablation needle assembly according to claim 6, **characterized in that** the ablation needle further comprises a drainage tube coaxially provided within the ablation needle body,
the ablation handle comprises an ablation needle body fixed seat and a drainage tube fixed seat, wherein a first cavity is formed at the gap where the proximal end of the ablation needle body and the ablation needle body fixed seat cooperate, and a second cavity is formed at the gap where a proximal end of the drainage tube and the drainage tube fixed seat cooperate; and
the first cavity is in communication with the cooling medium outlet channel, the cooling medium outlet channel being at least partially located within the ablation needle body fixed seat; and the second cavity is in communication with the cooling medium inlet channel, the cooling medium inlet channel being at least partially located within the drainage tube fixed seat.

8. The ablation needle assembly according to claim 7, **characterized in that** the drainage tube is provided therein with a second inner cavity extending from the proximal end to the distal end, the second inner cavity forming a second cooling medium flow path, and the second cooling medium flow path being in communication with the second cavity; and
the ablation needle body is provided therein with a first inner cavity extending from the proximal end to the distal end, and a gap between the first inner cavity and the drainage tube forms a first cooling medium flow path, the first cooling medium flow path being in communication with the first cavity.

9. The ablation needle assembly according to claim 8, **characterized in that** a portion of the ablation needle body extending out of the insulated coaxial outer tube has a cooling cavity inside, and the cooling cavity is in communication with both the first cooling medium flow path and the second cooling medium flow path.

10. The ablation needle assembly according to any one of claims 1 to 9, **characterized in that** the ablation needle assembly further comprises a biopsy needle and/or a puncture needle, the biopsy needle and the ablation needle are alternately inserted into the insulated coaxial outer tube and detachably connected to the insulated coaxial outer tube; and
the puncture needle and the ablation needle are alternately inserted within the insulated coaxial outer tube and detachably connected to the insulated coaxial outer tube, with a distal end of the puncture needle extending out of the insulated coaxial outer tube.

11. An ablation system, **characterized by** comprising the ablation needle assembly of any one of claims 1 to 10 and an energy generating device electrically connected to the ablation needle body of the ablation needle.
